# EUROPEAN PATENT APPLICATION

(11) **EP 4 350 486 A1**
(43) Date of publication of application: **10.04.2024**
(21) Application number: 23202180.8
(22) Date of filing: 06.10.2023
(51) Int. Cl.: G06F 3/01

(54) **EYE MONITORING SYSTEMS FOR DISTRESS MONITORING**

(30) Priority: 07.10.2022 US 202217962306
(71) Applicant: Hamilton Sundstrand Corporation, Charlotte, NC 28217-4578 (US)
(72) Inventor: LUKER, Kelly, Glastonbury, CT 06033 (US); ROHRIG, Jake, Simsbury, CT 06070 (US)
(74) Representative: Dehns

(57) **Abstract**

An eye monitoring system for distress monitoring can include an eye tracking device (101) configured to monitor one or more of an eye position, an eye movement, and/or an eye quality of a user, and to output eye tracking data. The system can also include a distress monitoring module (103) configured to receive the eye tracking data from the eye tracking device (101) and to determine whether the user is in distress based on the eye tracking data (e.g., in real time). The distress monitoring module (103) can be configured to output a distress signal if the user is determined to be in distress.

## Description

### FIELD

This disclosure relates to eye tracking systems.

### BACKGROUND

Currently, on spacewalks, astronaut distress is detected by lack of verbal communication between the astronaut and the command center. Additionally, during astronaut training, distress is detected by verbal communication (or lack thereof), and/or visual cues seen from the suit engineer or diver running the test. Detection in this way in either scenario is problematic because it takes time to identify distress, which in certain medical scenarios can be the difference between survival outcomes for the astronauts. There is also a subjective assessment required as to whether the scenario constitutes medical distress.

For instance, when an astronaut is training underwater, their medical distress is detected by either the diver looking at them or a lack of communication to command center. Astronaut medical distress detection is thus inefficient and subjective.

Such conventional methods and systems have generally been considered satisfactory for their intended purpose. However, there is still a need in the art for improvements. The present disclosure provides a solution for this need.

### SUMMARY

An eye monitoring system for distress monitoring can include an eye tracking device configured to monitor one or more of an eye position (e.g., gaze direction information), an eye movement (e.g., speed and/or pattern of movement), and/or an eye quality (e.g., pupil size) of a user, and to output eye tracking data. The system can also include a distress monitoring module configured to receive the eye tracking data from the eye tracking device and to determine whether the user is in distress based on the eye tracking data (e.g., in real time). The distress monitoring module can be configured to output a distress signal if the user is determined to be in distress.

In certain embodiments, the system can include a transmission module operatively connected to the distress monitoring module to receive the distress signal. In certain embodiments, the transmission module can be a radio configured to output the distress signal as a radio signal. In certain embodiments, the distress signal includes a spoken phrase indicating the user is in distress.

In certain embodiments, the distress monitoring module can be configured to determine a type of distress. In such embodiments, the distress signal can include an indication of the type of distress. In certain embodiments, the distress signal can include autonomous vehicle instructions configured to allow an autonomous vehicle to bring the user to a base location.

In certain embodiments, the eye tracking device can be mounted to a head worn device such that the eye tracking device is fixed relative to a head position of the user. For example, in certain embodiments, the head worn device includes glasses.

In certain embodiments, the eye tracking device can be a helmet mounted device such that the users head moves relative to the eye tracking device and the helmet. In certain embodiments, the helmet can be a space suit helmet. The eye tracking device can be mounted on an inside or outside of the helmet, for example.

In certain embodiments, the eye tracking device can be a contact lens sensor device configured to be worn on an eye and to wirelessly transmit eye tracking data to the distress monitoring module. The contact lens sensor device can include any suitable sensors to track one or more eye qualities and/or movement (e.g., MEMS accelerometers). Any other suitable eye tracking device is contemplated herein.

In accordance with at least one aspect of this disclosure, a hazardous environment suit assembly can include an air tight suit configured to enclose a user in an air tight environment. The assembly can also include an eye monitoring system for distress monitoring, e.g., as disclosed herein, e.g., as described above. Certain embodiments of the assembly can include any suitable components of an eye monitoring system as disclosed herein, e.g., as described above.

In certain embodiments, the air tight suit can be a space suit. Any other suitable suit (e.g., a firefighters suit, a hazmat suit, etc.) and/or application of the suit is contemplated herein.

In accordance with at least one aspect of this disclosure, non-transitory computer readable medium, comprising computer executable instructions configured to cause a computer to perform a method. The method can include monitoring eye tracking data of a user in an air tight suit, determining if the user is in medical distress based on the eye tracking data, and outputting a distress signal to indicate distress. Outputting the distress signal can include outputting a radio signal indicating the user in distress and the distress type if determined. In certain embodiments, the method can include utilizing one or more auxiliary biometrics along with the eye tracking data to determine a medical distress condition.

These and other features of the embodiments of the subject disclosure will become more readily apparent to those skilled in the art from the following detailed description taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, embodiments thereof will be described in detail herein below with reference to certain figures, wherein:
Fig. 1 is a schematic diagram of an embodiment of an eye monitoring system and suit assembly in accordance with this disclosure;
Fig. 2 is a schematic diagram of an embodiment of an eye monitoring system and suit assembly in accordance with this disclosure; and
Fig. 3 is a schematic diagram of an embodiment of an eye monitoring system and suit assembly in accordance with this disclosure.

### DETAILED DESCRIPTION

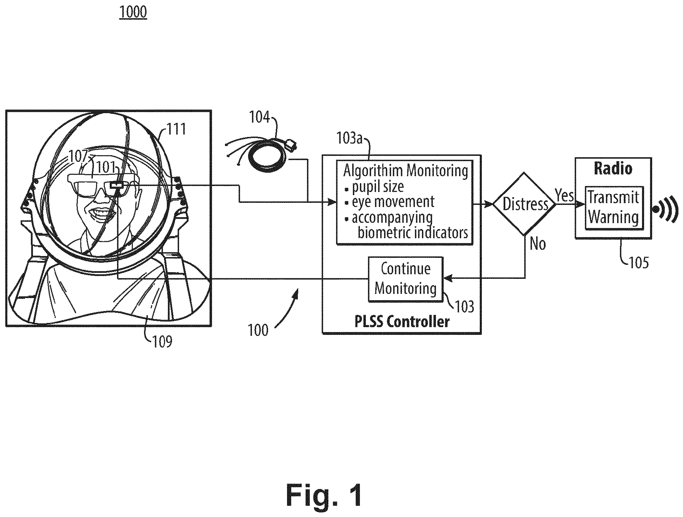

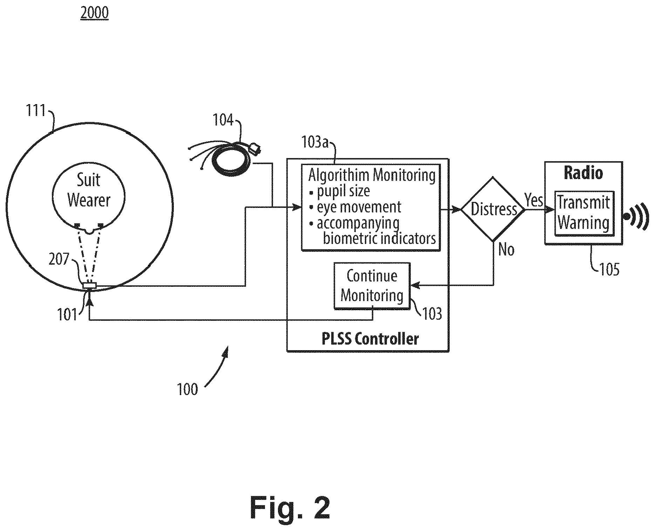

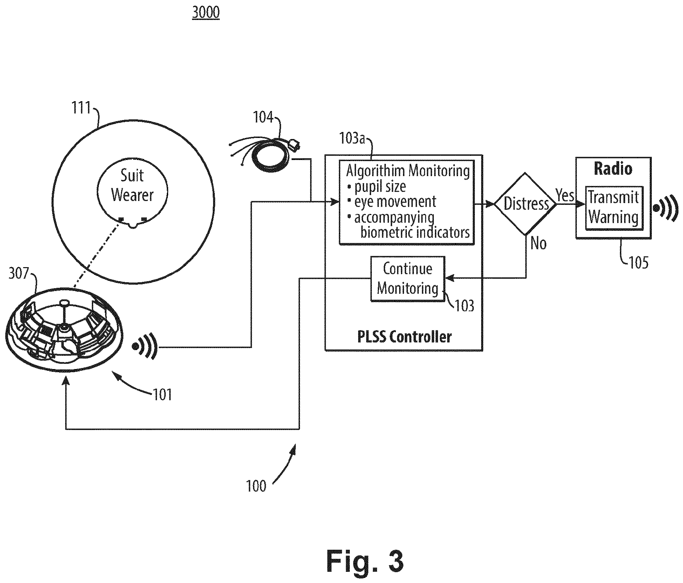

Reference will now be made to the drawings wherein like reference numerals identify similar structural features or aspects of the subject disclosure. For purposes of explanation and illustration, and not limitation, an illustrative view of an embodiment of a system in accordance with the disclosure is shown in Fig. 1 and is designated generally by reference character 100. Other embodiments and/or aspects of this disclosure are shown in Figs. 2 and 3. Certain embodiments described herein can be used to provide health monitoring for certain high risk personnel (e.g., astronauts, first responders, firemen, hazmat personnel, pilots).

Referring to Fig. 1, an eye monitoring system 100 for distress monitoring can include an eye tracking device 101 configured to monitor one or more of an eye position (e.g., gaze direction information), an eye movement (e.g., speed and/or pattern of movement), and/or an eye quality (e.g., pupil size) of a user, and to output eye tracking data. For example, the eye tracking device 101 can be configured to determine a pupil size, a change in pupil size, eye coloration, direction of gaze, rapid eye movements/vibration, and/or any other suitable medically relevant factors relating to the eye. Any suitable number of eye tracking devices in a system 100 is contemplated herein.

The system 100 can also include a distress monitoring module 103 configured to receive the eye tracking data from the eye tracking device 101 and to determine whether the user is in distress based on the eye tracking data (e.g., in real time). In certain embodiments, the distress monitoring module 103 can be embodied as any suitable software and/or hardware component of a Portable Life Support System (PLSS) (e.g., of a space suit). The distress monitoring module 103 can be configured to output a distress signal if the user is determined to be in distress (e.g., hypoxia). The distress module 103 can include any suitable hardware and/or software module(s) configured to evaluate the eye tracking data and determine whether a user is in distress. For example, the distress monitoring module 103 can include a health monitoring logic module 103a configured to utilize any suitable logic (e.g., artificial intelligence/machine learning) to correlate the eye tracking data to one or more medical conditions.

In certain embodiments, the system 100 can include auxiliary health monitoring equipment 104 (e.g., an ECG/EKG system, pulse oximeter, etc.) to output user health data, for example. Any suitable type of health monitoring equipment is contemplated herein. The distress monitoring module 103 can be operatively connected to the auxiliary health monitoring equipment 104 to receive the user health data. The distress monitoring module 103 can be configured to use the user health data with or separate from the eye tracking data to determine whether the user is in a distressed state. In some cases, any independent data pathway can be used to correlate with one or more other data pathways to make a more accurate conclusion as to the medical state of the user (e.g., for determining whether there is actually distress and/or classifying the distress type).

In certain embodiments, the system 100 can include a transmission module 105 operatively connected to the distress monitoring module 103 to receive the distress signal. As functionally shown, if the distress monitoring module 103 determines there to be distress, it can send the distress signal to the transmission module 105, and/or otherwise continue monitoring. In certain embodiments, the transmission module 105 can be a radio, e.g., as shown, configured to output the distress signal as a radio signal. In certain embodiments, the distress signal can include a spoken phrase indicating the user is in distress (e.g., a machine spoken statement such as "Buzz is in distress"). Any suitable type of radio signal, e.g., audible, inaudible, pattern of beeps, coded, etc., to signal any distress or type of distress is contemplated herein.

In certain embodiments, the distress monitoring module 103 can be configured to determine a type of distress (e.g., the specific medical condition). In such embodiments, the distress signal can include an indication of the type of distress. For example, in embodiments where the distress signal can include a spoken phrase indicating the user is in distress, the phrase can include the specific medical condition or other type of distress (e.g., a machine spoken statement such as "Buzz is hypoxic").

In certain embodiments, the distress signal can include autonomous vehicle instructions configured to allow an autonomous vehicle (not shown) to bring the user to a base location. This can be useful for astronauts, for example, on planetary missions where a rover can automatically retrieve the astronaut and bring them to safety. A similar scheme can be used for any other suitable application (e.g., firefighters, hazmat personnel, etc.).

In certain embodiments, e.g., as shown in the assembly 1000 of Fig. 1, the eye tracking device 101 can be mounted to a head worn device 107 such that the eye tracking device 101 is fixed relative to a head position of the user. For example, in certain embodiments, the head worn device 107 includes glasses, e.g., as shown. The head worn device 107 can be configured to be worn within a sealed environment of an air tight suit 109 (e.g., within a helmet 111 of the suit 109).

In certain embodiments, e.g., as shown in assembly 2000 of Fig. 2, the eye tracking device 101 can be a helmet mounted device 207 such that the users head moved relative to the eye tracking device(s) and the helmet. In certain embodiments, the helmet can be a space suit helmet. The eye tracking device 101 can be mounted on an inside or outside of the helmet 111, for example. In certain embodiments, a camera or other eye tracking device can be located outside the helmet looking in, for example (e.g., bonded to the helmet). In certain embodiments and applications, having such equipment outside of the internal oxygen environment of the suit can be advantageous. Any suitable number of eye tracking devices 207 is contemplated herein.

In certain embodiments, e.g., as shown in assembly 3000 of Fig. 3, the eye tracking device 101 can be a contact lens sensor device 307 configured to be worn on an eye and to wirelessly transmit eye tracking data to the distress monitoring module 103. The contact lens sensor device 307 can include any suitable sensors to track one or more eye qualities and/or movement (e.g., MEMS accelerometers). Any other suitable eye tracking device is contemplated herein.

In accordance with at least one aspect of this disclosure, a hazardous environment suit assembly (e.g., assemblies 1000, 2000, 3000) can include an air tight suit (e.g., 109 as shown in Fig. 1) configured to enclose a user in an air tight environment. The assembly 1000, 2000, 3000 can also include an eye monitoring system 100 for distress monitoring, e.g., as disclosed herein, e.g., as described above. Certain embodiments of the assembly 1000, 2000, 3000 can include any suitable components of an eye monitoring system 100 as disclosed herein, e.g., as described above.

In certain embodiments, the air tight suit 109 can be a space suit for an astronaut, for example. Any other suitable suit (e.g., a firefighter's suit, a hazmat suit, etc.) and/or application of the suit is contemplated herein.

In accordance with at least one aspect of this disclosure, non-transitory computer readable medium, comprising computer executable instructions configured to cause a computer to perform a method. The method can include monitoring eye tracking data of a user in an air tight suit, determining if the user is in medical distress based on the eye tracking data, and outputting a distress signal to indicate distress. Outputting the distress signal can include outputting a radio signal indicating the user in distress and the distress type if determined. In certain embodiments, the method can include utilizing one or more auxiliary biometrics (e.g., from device 104) along with the eye tracking data to determine a medical distress condition.

Embodiments can be used for in suit health monitoring for distress scenarios, for example. For example, an eye monitoring system can detect pupil dilation (which is an indicator for hypoxia), e.g., by seeing light reflect from the back of an eye (e.g., more light being reflected from a more dilated pupil). Embodiments can compare pupil size to a known size or average size of the user, for example. Embodiments can utilize computer vision for eye tracking (e.g., using image comparison) instead or additionally, for example, such that the eye tracking device includes a camera and the eye tracking data includes image information.

Embodiments can automatically monitor and can output a radio signal (e.g., a spoken phrase, an error code seen by ground personnel and/or other astronauts, etc.) that the user is in distress. Embodiments can include a visual cue of distress (e.g., a red light on visible from outside the suit). Certain embodiments can send a command to a rover to take the user back to a habitat or other location that is safe.

Certain embodiments can be head mounted and thus not need to account for head movement. Certain embodiments can be mounted inside helmet that is fixed relative to a user's head. Such embodiments can account for face and head position while looking at a position of the eyes and/or other eye information. Certain embodiments can utilize computer vision and/or any other suitable processing to account for head motion data. Certain embodiments can use any suitable head position determination system (e.g., wearable or rear helmet mounted optical fiducials such as balls or any other suitable shape or reference). A fiducial marker or optical fiducial can include an object placed in the field of view of an imaging system that appears in the image produced, for use as a point of reference or a measure. In certain embodiments, QR codes can be used as fiducials. Any suitable consistent, distinguishable markers that a computer can recognize and locate with computer vision are contemplated herein. Certain embodiments can be on-eye contacts that have the ability to broadcast information about the eye. Any other suitable eye tracking system is contemplated herein.

Embodiments can utilize eye tracking for medical distress. Certain embodiments can monitor pupil size, eye movement, and/or other accompanying biometric indicators, for example. Certain embodiments involve incorporating eye tracking technology into either an on-eye, head-worn, or off-head system for the purpose of medical distress detection. In certain embodiments, an example of eye tracking technology utilizes the reflection of infrared light to filter and calculate where you are looking. As eye tracking measurement technology evolves, the eye tracking technology may function differently.

Common symptoms of medical distress, e.g., in astronauts in space, can include eyes rolling behind the head, squinting eyes/face, zoning out/not blinking, and pupil dilation. Certain embodiments can utilize the reflection of infrared light to filter and calculate where a user is looking, for example. Certain embodiments can track these symptoms via eye movement because not every astronaut will use verbal cues to indicate a medical emergency. In certain embodiments, through continuous monitoring, a test lead or fellow astronaut can be quickly alerted when certain eye expressions are held too long or are occurring. In certain embodiments, auxiliary sensors can be used to further isolate the medical condition (e.g., accelerometers accompanying eye tracking to indicate a seizure).

Embodiments can eliminate subjective human assessments to determine a medical emergency, giving consistent results every time. Embodiments can provide a faster reaction time which can therefore increase favorable outcomes during a medical emergency. Determining lack of communication from the astronaut and declaring a course of action can take time in a situation where 30-60 seconds of oxygen deprivation can cause a loss of consciousness and 60 or more seconds can cause brain cell death. Certain embodiments can reduce the response time in detecting and declaring medical emergency which increases treatment time.

Certain embodiments can enables more range between crew as members no longer need to visibly assess partner health. Certain embodiments can allow elimination of unnecessary verbal or visual confirmation of health on EVAs and/or training (e.g., Mission Control or divers assessing health respectively). Certain embodiments can be applicable to fighter pilots and any other suitable areas (e.g., NASA Divers, firefighters, medical patients, e.g., subjects in MRI machines).

Certain embodiments increase the automated/advanced technology that increases crewmember safety. Such increases help deep space exploration and long-term missions become more achievable.

Certain embodiments can include any suitable computer hardware and/or software module(s) to perform any suitable function (e.g., as disclosed herein). As will be appreciated by those skilled in the art, aspects of the present disclosure may be embodied as a system, method or computer program product. Accordingly, aspects of this disclosure may take the form of an entirely hardware embodiment, an entirely software embodiment (including firmware, resident software, micro-code, etc.), or an embodiment combining software and hardware aspects, all possibilities of which can be referred to herein as a "circuit," "module," or "system." A "circuit," "module," or "system" can include one or more portions of one or more separate physical hardware and/or software components that can together perform the disclosed function of the "circuit," "module," or "system", or a "circuit," "module," or "system" can be a single self-contained unit (e.g., of hardware and/or software). Furthermore, aspects of this disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out operations for aspects of this disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of this disclosure may be described above with reference to flowchart illustrations and/or block diagrams of methods, apparatus (systems) and computer program products according to embodiments of this disclosure. It will be understood that each block of any flowchart illustrations and/or block diagrams, and combinations of blocks in any flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in any flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Those having ordinary skill in the art understand that any numerical values disclosed herein can be exact values or can be values within a range. Further, any terms of approximation (e.g., "about", "approximately", "around") used in this disclosure can mean the stated value within a range. For example, in certain embodiments, the range can be within (plus or minus) 20%, or within 10%, or within 5%, or within 2%, or within any other suitable percentage or number as appreciated by those having ordinary skill in the art (e.g., for known tolerance limits or error ranges).

The articles "a", "an", and "the" as used herein and in the appended claims are used herein to refer to one or to more than one (i.e., to at least one) of the grammatical object of the article unless the context clearly indicates otherwise. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

Any suitable combination(s) of any disclosed embodiments and/or any suitable portion(s) thereof are contemplated herein as appreciated by those having ordinary skill in the art in view of this disclosure.

The embodiments of the present disclosure, as described above and shown in the drawings, provide for improvement in the art to which they pertain. While the subject disclosure includes reference to certain embodiments, those skilled in the art will readily appreciate that changes and/or modifications may be made thereto without departing from the scope of the subject disclosure.

## Claims

1. An eye monitoring system for distress monitoring, comprising:
an eye tracking device (101) configured to monitor one or more of an eye position, an eye movement, and/or an eye quality of a user, and to output eye tracking data; and
a distress monitoring module (103) configured to receive the eye tracking data from the eye tracking device (101) and to determine whether the user is in distress based on the eye tracking data, wherein the distress monitoring module (103) is configured to output a distress signal if the user is determined to be in distress.

2. The system of claim 1, wherein the system includes a transmission module (105) operatively connected to the distress monitoring module (103) to receive the distress signal.

3. The system of claim 2, wherein the transmission module (105) is a radio configured to output the distress signal as a radio signal.

4. The system of claim 3, wherein the distress signal includes a spoken phrase indicating the user is in distress.

5. The system of any preceding claim, wherein the distress monitoring module (103) is configured to determine a type of distress, wherein the distress signal includes an indication of the type of distress.

6. The system of any preceding claim, wherein the distress signal includes autonomous vehicle instructions configured to allow an autonomous vehicle to bring the user to a base location.

7. The system of any preceding claim, wherein the eye tracking device (101) is mounted to a head worn device (107) such that the eye tracking device is fixed relative to a head position of the user.

8. The system of claim 7, wherein the head worn device (107) includes glasses.

9. The system of any preceding claim, wherein the eye tracking device (101) is a helmet mounted device (207) such that the users head moved relative to the eye tracking device (101) and the helmet.

10. The system of claim 9, wherein the helmet is a space suit helmet, wherein the eye tracking device (101) is mounted on an inside or an outside of the helmet.

11. The system of any preceding claim 1, wherein the eye tracking device (101) is a contact lens sensor device (307) configured to be worn on an eye and to wirelessly transmit eye tracking data to the distress monitoring module.

12. A hazardous environment suit assembly, comprising:
an air tight suit (109) configured to enclose a user in an air tight environment; and
an eye monitoring system of any preceding claim.

13. The assembly of claim 12, wherein the air tight suit (109) is a space suit.

14. A non-transitory computer readable medium, comprising computer executable instructions configured to cause a computer to perform a method, the method comprising:
monitoring eye tracking data of a user in an air tight suit;
determining if the user is in medical distress based on the eye tracking data; and
outputting a distress signal to indicate distress.

15. The non-transitory computer readable medium of claim 14, wherein outputting the distress signal includes outputting a radio signal indicating the user in distress and the distress type if determined, or utilizing one or more auxiliary biometrics along with the eye tracking data to determine a medical distress condition.
